# EUROPEAN PATENT APPLICATION

(11) **EP 1 921 553 A1**
(43) Date of publication of application: **14.05.2008**
(21) Application number: 07120033.1
(22) Date of filing: 06.11.2007
(51) Int. Cl.: G06F 19/00

(54) **Control of drug administration**

(30) Priority: 08.11.2006 US 557635
(71) Applicant: General Electric Company, Schenectady, NY 12345 (US)
(72) Inventor: Vierto-Oja, Hanna E., 02660 Espoo (FI)
(74) Representative: Bedford, Grant Richard

(57) **Abstract**

The invention relates to a method and apparatus (500) for controlling drug administration. In order to achieve an easy-to-use and technically reliable control mechanism that can be implemented cost-effectively, input information including patient information and effect level infonnation for at least one type of drug effect to be achieved in a patient is supplied to the control mechanism. Synergetic pharmacodynamic models are utilized to determine, based on the input information, effect-site concentration(s) for at least one drug to be administered. A pharmacokinetic model of each drug may then be utilized to define, based on the effect-site concentration of the drug, a delivery rate for the drug to be administered.

## Description

The present invention relates generally to control of drug administration. The invention finds a typical application in anesthesia, where a plurality of drugs of different types is simultaneously administered to the patient.

A drug delivery system typically consists of a drug delivery unit and a drug delivery controller. The drug delivery unit is a mechanical arrangement that delivers drugs to a patient. It may be, for example, a manually or actuator operated syringe, a volume controlled infusion pump, or an evaporator with an anesthesia gas circuit in an anesthesia machine. A drug delivery controller in turn refers to a set-up, which may be based on an automated program, an algorithm, or on a decision-making or support tool, which adjusts the drug dose or concentration to a desired level for the patient.

A pharmacokinetic model describes how the drug is distributed in the course of time from the site of delivery to different parts of the body and to the particular organ, such as the brain, in which the drug is supposed to have its effect. A pharmacokinetic model comprises of a set of mathematical differential equations that describes the distribution of a drug within the body as a function of time. The model includes both drug-specific and patient-specific parameters. The drug-specific parameters describe the diffusion and solubility properties of the drug, whereas the patient-specific parameters describe the properties of the patient that affect the drug distribution, such as weight, height, age, and gender. The body of the patient is represented by a simplified system of typically four tissue compartments among which the drug is distributed according to the differential equations.

In target controlled infusion (TCI) the operator sets a target concentration of the drug for the patient and an automated controller drives the concentration to the target level by optimally adjusting the infusion rate of a pump. A target-controlled infusion (TCI) pump helps the clinician in controlling the administration of drugs by incorporating a pharmacokinetic (Pk) model in the controlling logic. With a TCI pump, a clinician can therefore directly set the desired effect-site concentration for the target organ, and the device calculates the infusion rate needed to reach and maintain that particular concentration based on a (typically three) tissue compartment model using information on the weight and the height of the individual patient. The pump typically includes a display that shows the infusion rate, the estimated plasma concentration, and the estimated effect-site concentration.

Even equipped with a TCI pump, however, a clinician still needs to memorize and understand a set of complicated issues. The potencies of different drugs vary widely: for propofol, which is a hypnotic agent, typical effect-site concentrations, for example, are measured in units of micrograms per milliliter, whereas for remifentanil, which is an opioid typically used in anesthesia, the concentrations need to be measured in nanograms per milliliter. The required effect-site concentrations are different for each hypnotic and opioid, and the use of volatiles obviously adds to the complexity. A further significant complication arises from the strong synergetic relation between hypnotics and opioids: adding of opioid dramatically increases the effective potency of the hypnotic drug, and vice versa. This makes it more difficult for the clinician to independently control the hypnotic and analgesic components of anesthesia in a predefined manner.

Consequently, a major drawback related to the use of TCI pumps is the complexity of the drug administration process. Although the control logic of a TCI pump is provided with a pharmacokinetic model, a clinician has no technical facilities for assessing the synergetic effects of the drugs administered and the pharmocodynamics involved, despite the fact that pharmacodynamic models already exist.

Pharmacodynamic models are statistical models that describe the relationship between the effect-site concentration and the resulting effect. For example, the pharmacodynamic model of a hypnotic indicates the effect-site concentration at which a certain proportion, such as 50 or 90 per cent, of patients loses consciousness or is in an unconscious state of a certain level. Recently, pharmacodynamic models for certain drug combinations have been developed, which incorporate the interaction effect of a hypnotic drug and an opioid, cf. Bouillon et al.: Pharmacodynamic Interaction between Propofol and Remifentanil Regarding Hypnosis, Tolerance of Laryngoscopy; Bispectral Index, and Electroencephalographic Approximate Entropy, Anesthesiology 2004; 100: 1353-72, or Kern et al.: Opioid-Hypnotic Synergy, Anesthesiology 2004; 100: 1373-81. Applying such models, one can estimate the hypnotic and analgesic effects of a particular drug combination. Although existing models concern only certain drugs, they will presumably cover most of the typical anesthesia regime in the very near future.

At present, some operation theaters provided with advanced technology may already be equipped with a drug delivery system capable of utilizing both pharmacokinetic and synergetic pharmacodynamic models.

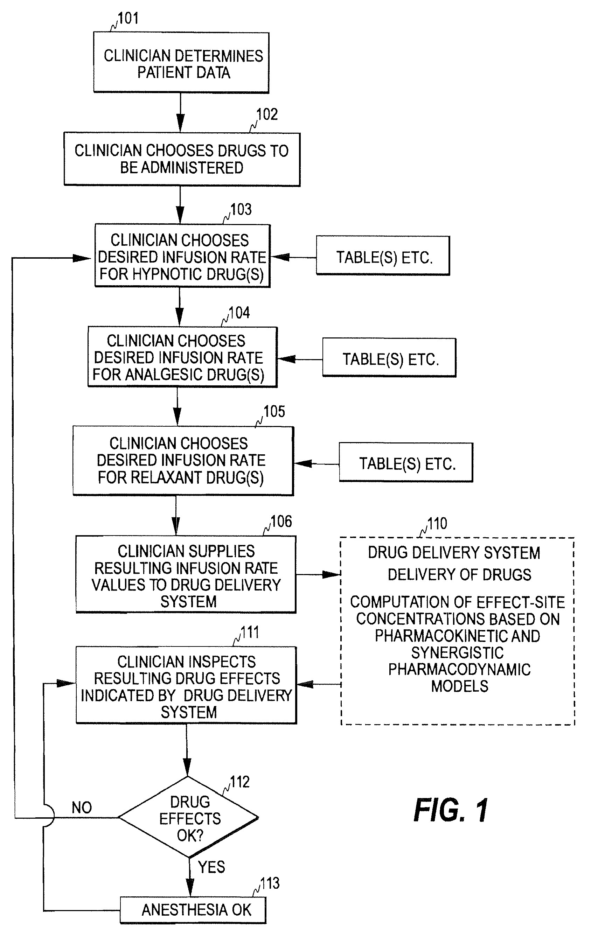

Next, the operation of such a system is discussed with reference to FIG. 1, which is a flow diagram illustrating an example of the control of the administration of anesthetics in connection with a prior art system provided with pharmacokinetic and synergetic pharmacodynamic models. In this system, anesthesia is controlled by an anesthesiologist and the drug delivery system provides guiding information assisting the anesthesiologist in his/her decisions. For the anesthesia, a clinician first determines patient data, such as age, weight, height, and gender and enters the data into the system. The clinician then selects the drugs to be administered, which include hypnotic and analgesic drugs, and typically also a relaxant drug (steps 101 and 102). Based on the patient data, the clinician then chooses desired infusion rates for the drugs (steps 103 to 105). For this, the clinician may utilize various assisting tools, such as tables, compiled for a wide range of drugs and patients. The clinician then supplies (step 106) the infusion rates to a drug delivery system 110 which delivers the drugs and calculates the effect-site concentrations for the selected drugs based on the pharmacokinetic models and the effects of the selected drugs based on the effect-site concentrations and the synergistic pharmacodynamic models stored in the system. The delivery system displays the resulting effect-site concentrations and drug effects during the complete course of the anesthesia (step 111). When the clinician, utilizing the presented information, at any instant recognizes a need to change the chosen drug administration rates, (s)he may return to step 103 to select new infusion rates for the drugs and the above preparatory steps are carried out again to check if the new infusion rates result in desired drug effects. The drug effects are displayed together with a statistical curve indicating how large a proportion of patients would response to a certain stimulus, such as speech or laryngoscopy, at a particular drug effect level.

A system of the above type is marketed under trade name Carestation^{™} by GE Healthcare, General Electric Company, US.

It has also been suggested that the administration of a hypnotic drug could be controlled in a closed loop fashion. U.S. Patent 6,631,291 B2 discloses a system in which a quantification of the complexity of the EEG signal data measured from the patient is used to determine the patient's hypnotic level and to control the administration of a hypnotic drug to the patient in a closed loop fashion. A closed loop drug administration system generally refers to a system in which the administration is controlled automatically based on feedback measurement data measured from the patient, whereas an open loop drug administration system refers to a system in which such feedback measurement data is not automatically utilized for the control of drug administration.

Furthermore, U.S. Patent Application 2006/0217628 (Applicant's case 862) discloses a closed loop system in which an index of hypnosis and an index of nociception are measured from the patient and supplied to a control unit. The control unit determines the location of patient state on a two-dimensional plot. The control unit also compares the determined location with the input parameters defining the targeted location and controls the anesthetic delivery system to shift the state towards the targeted state or to maintain the state of the patient in the targeted domain.

There are, however, many technical difficulties in designing a well-performing closed loop system. This is partly due to the complexity of the human body, which makes the measurement of reliable and accurate feedback information from the patient complex. Secondly, the measurements are not completely tolerant to external interference: see for example White et al., "A Comparison of State and Response Entropy Versus Bispectral Index Values During the Perioperative Period", Anaesth Analg 2006; 102:160-7, which discusses the effect of electrocautery on some commercial measurements of the hypnotic component of anesthesia. If a measurement is not working correctly due to an external artifact, there is a serious risk that either too much or too little drug is administered to the patient. An experienced anesthesiologist can usually interpret these situations correctly, by utilizing the information of what is actually taking place in the operation room at the time. Moreover, an automated computer program has many interrelated device and patient parameters, which should be known in order to achieve a timely and quantitatively precise delivery of right drugs at right instants and in right doses for each patient. Due to the above problems, the cost-effectiveness of closed loop systems have not yet matured to a level enabling commercial use, and therefore FDA (U.S. Food and Drug Administration) approved automated closed loop systems do not exist at present.

The present invention seeks to improve the existing control mechanisms for drug administration and to accomplish a new solution for controlling the administration of drugs.

Various aspects of the present invention seek to accomplish an improved drug control mechanism for controlling drug delivery in a clinical environment, where a set of drugs with synergetic effects may be administered to a patient. Various aspects of the present invention further seek to accomplish a drug control mechanism which is easy-to-use, technically reliable and can be implemented cost-effectively without resorting to the rather complicated feedback measurements from the patient.

In various aspects of the present invention, the control of drug administration is performed without measuring feedback information from the patient, by supplying the desired level(s) of at least one type of drug effect as input information and computing the drug delivery rate(s) based on pharmacokinetic and synergetic pharmacodynamic models. The drug effect here refers to the effect that the drug concerned will have on the patient in statistical sense, and the drug effect level may simply be a value on a predetermined scale. For example, for the hypnotic effect the clinician may select a level at which 90 per cent of the patients within the same patient group lose consciousness.

As discussed above, the relationship between drug effect and drug concentration is defined by a pharmacodynamic model, whereby the model allows the determination of the effect-site concentration based on the effect. However, it is to be noted that there is no such direct correspondence between the drug effect computed from a pharmacokinetic/dynamic model and the effect measured from the patient in clinical environment. For example, the hypnotic level measured from the patient is affected by the current stimulation of the patient and therefore a measure of the hypnotic level derived from the patient also incorporates the effect of stimulation.

Thus one aspect of the invention is providing a method for controlling drug administration. The method comprises receiving input information for drug administration, wherein the input information includes patient information and effect level information for at least one type of drug effect to be achieved in a patient. The method further comprises defining, based on the input information and at least one pharmacodynamic model, an effect-site concentration for a drug to be administered.

The internal logic of certain embodiments of the present invention is thus opposite to the above-described prior art systems provided with pharmacokinetic and pharmacodynamic models: the user inputs the desired level for at least one type of drug effect and the system determines the delivery rates needed to achieve the desired level. In this way the amount of complicated information that needs to be remembered and processed by the clinician may be significantly reduced. Furthermore, the problems related to the implementation of a reliably operating closed loop system may be avoided, since the control does not require feedback information from the patient.

Another aspect of the invention is that of providing an apparatus for controlling drug administration. The apparatus comprises a user interface module configured to receive input information for drug administration, wherein the input information includes patient information and effect level information for at least one type of drug effect to be achieved in a patient. The apparatus further comprises a computation module configured to define, based on the input information and at least one pharmacodynamic model, an effect-site concentration for a drug to be administered.

A further aspect of the invention is that of providing a computer program product for controlling drug administration. The program product includes a first program code portion configured to receive input information for drug administration, wherein the input information includes patient information and effect level information for at least one type of drug effect to be achieved in a patient. The computer product further comprises a second program code portion configured to define, based on the input information and at least one pharmacodynamic model, an effect-site concentration for a drug to be administered.

Various features and advantages of the invention will become apparent by reference to the following detailed description and accompanying drawings, in which
FIG. 1 is a flow diagram illustrating a prior art open-loop control mechanism for controlling the administration of anesthetics;
FIG. 2 is a flow diagram illustrating one embodiment of the control mechanism of the invention for controlling the administration of drugs;
FIG. 3 illustrates the selection of the drug effect levels according one embodiment of the invention;
FIG. 4 is a flow diagram illustrating one embodiment of the operation of the drug delivery system shown in FIG. 2; and
FIG. 5 illustrates one embodiment of a system according to the invention.

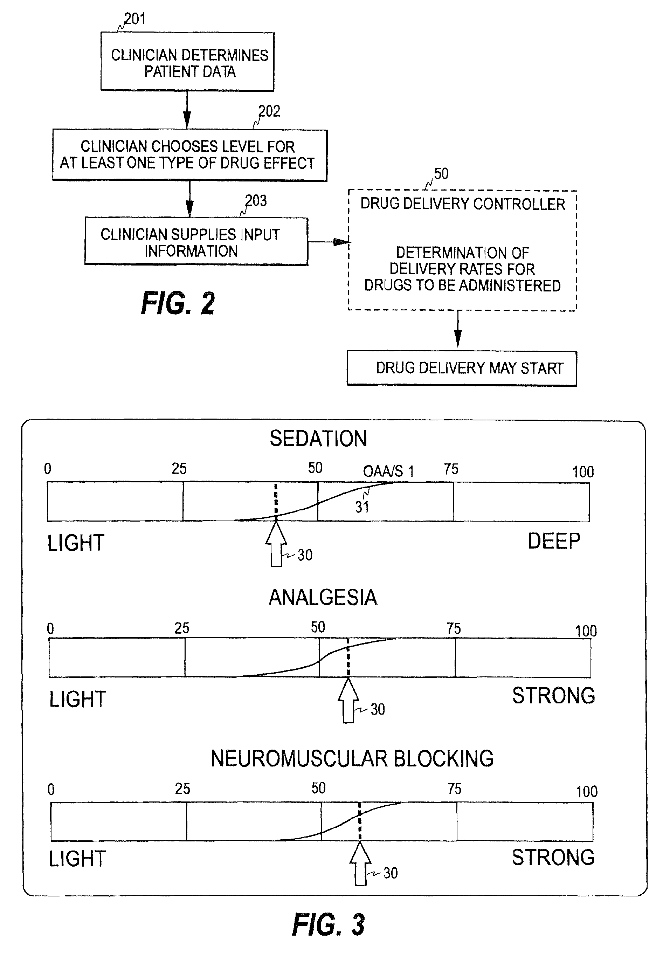

FIG. 2 illustrates one embodiment of the control mechanism of the invention. The clinician first determines patient data, such as age, weight, height, and gender (step 201). After this, the clinician selects a desired level for at least one type of drug effect at step 202. For example, for anesthesia the clinician typically selects a desired level for hypnotic drug effect and a desired level for analgesic drug effect, often also a desired level for relaxant drug effect.

Having selected the drug effect level(s), the clinician supplies the patient data and the selected effect level(s) as input information to a drug delivery controller 50 of the invention (step 203). As discussed below, the drug delivery controller may also assist the clinician in determining the patient data and selecting the effect level(s). Based on the input information, the drug delivery controller then determines a delivery rate for each drug to be administered. In the input information the clinician may also indicate the drugs to be used. However, this is not necessary, since the drug delivery controller may also select the drugs before determining the associated delivery rates. Upon determination of the delivery rates the system is ready to start the administration of the drugs.

FIG. 3 illustrates the selection of desired levels at step 202 according to one embodiment of the invention. It assumed in this example that the clinician may select desired levels for three types of drug effects: hypnotic effect, analgesic effect, and neuromuscular blocking effect. For the selection, a dedicated scale may be displayed for each effect type on the screen of a display unit of the drug delivery system. On each scale one end corresponds to a mild effect and the opposite end to a strong effect. By moving a pointer, such as the arrow 30 shown in the figure, on the scale, the clinician may choose a desired level for the corresponding drug effect.

Each scale may also be provided with a statistical reference curve 31 indicating how the proportion of patients responding in a certain manner increases as the effect level increases. The curve may be provided with a legend indicating the type of (un)responsiveness corresponding to the curve, such as unresponsiveness to a vocal stimulus. In one embodiment, the user may also select from the options of the menu of the user interface whether or not the reference curve is displayed in connection with the predetermined scale.

In the embodiment of FIG. 3, the clinician may thus select the desired level on a continuous scale for each drug effect type. In an alternative embodiment, the selection may be made from among a set of values or value ranges. For example, the scale may be divided into a desired number of ascending or descending steps, whereby the user may choose the step that corresponds to the effect level desired.

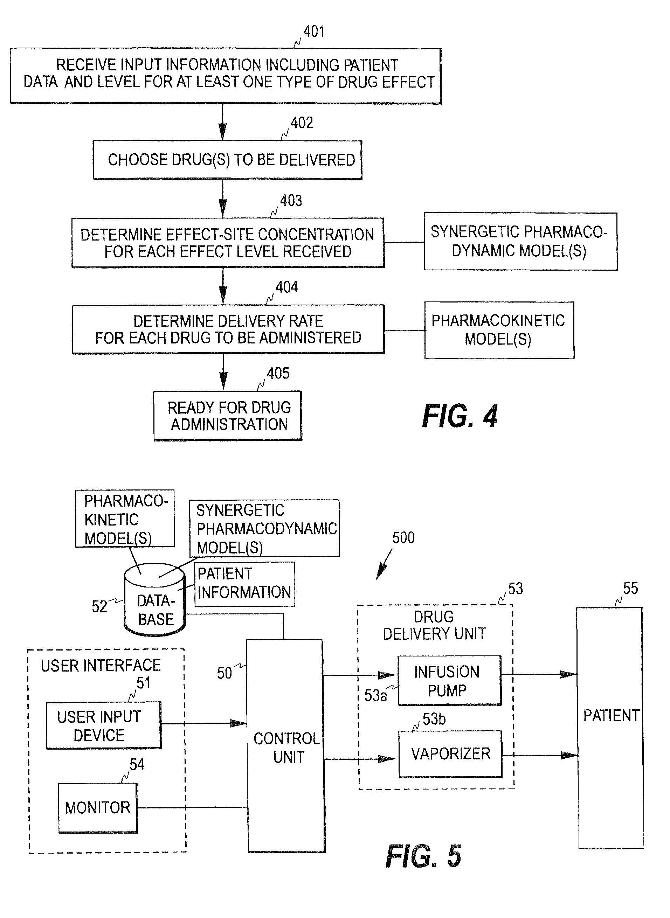

FIG. 4 illustrates the operation of the drug delivery controller 50 according to one embodiment of the invention. As discussed above, the input information supplied by the user includes at least patient data and the desired effect level for at least one type of drug effect. If the user does not indicate the drugs to be used, the system selects the drugs by which the desired effect levels are to be achieved (step 402). For each effect type, the system may use one or more drugs. Using the input information and synergetic pharmacodynamic models of the drugs to be used, i.e. pharmacodynamic models in which the synergetic effect of the drugs to be used is taken into account, the delivery controller then determines the effect-site concentrations for each drug to be administered (step 403). Pharmacokinetic models are then utilized at step 404 to determine, based on the effect-site concentration, a delivery rate for each drug to be administered. After this, the system is ready for drug administration.

FIG. 5 illustrates one embodiment of the drug delivery system 500 according to the invention. The core of the apparatus is a control unit 50, i.e. the drug delivery controller, which receives the input information supplied by a user through a user input device 51, which may include a keyboard, a mouse, and/or a bar code reader, for example. The user interface of the system further comprises a monitor 54 for displaying various information to the user and for enabling the user to select the desired effect level(s).

The control unit is provided with a memory unit or database 52 storing the input information and the pharmacokinetic and the synergetic pharmacodynamic models for the drugs that may be administered through a drug delivery unit 53.

The drug delivery unit is typically an anesthesia delivery unit, which may comprise an intravenous infusion pump 53a for intravenously administered drugs and/or a vaporizer 53b for inhaled drugs.

The control unit receives the input information through the user interface and defines the delivery rates based on the said information and the models stored in the database. The number and selection of the drugs available in the delivery unit may vary, and a practical and cost-effective implementation may include only one drug for each effect type.

The control unit may control the drug delivery unit directly based on the delivery rates determined or it may display the defined delivery rate(s) to the user and prompt the user to accept the commencement of drug administration.

Although one computer or processor unit may act as the control unit, the processing of the data may also be distributed among different units/processors (servers) within a network, such as a hospital LAN (local area network). The apparatus of various embodiments of the invention may thus also be implemented as a distributed system. For example, the control unit may send the input information to another processor unit provided with the models for determining the effect-site concentrations and the delivery rates.

The control unit may also be connected to a TCI pump, i.e. the infusion pump 53a of FIG. 5 may be a TCI pump. In this embodiment, the operation of the control unit in step 404 of FIG. 4 is modified so that in the said step the delivery rate is determined only for drugs to be administered through delivery units not provided with built-in pharmacokinetic models, whereas the determination of the delivery rate is carried out within a corresponding TCI pump for each drug to be administered through a TCI pump. The control unit supplies the effect-site concentration to the TCI pump concerned.

Although the above-described control mechanism is mainly intended for anesthesia, it may also be used in intensive care units, for example. The mechanism may also be utilized to control only one type of drug effect. In an uncomplex implementation, the control mechanism may thus include a control unit determining an effect-site concentration for a single TCI pump. However, the more the potencies of the drugs vary and the more complicated the synergetic effects are, the greater is the benefit achieved through the invention.

Although the invention was described above with reference to the examples shown in the appended drawings, it is obvious that the invention is not limited to these, but may be modified by those skilled in the art without departing from the scope and spirit of the invention.

Various aspects and embodiments of the present invention are now defined in the following numbered clauses:
1. A method for controlling drug administration, the method comprising:
   - receiving input information for drug administration, wherein the input information includes patient information and effect level information for at least one type of drug effect to be achieved in a patient; and
   - defining, based on the input information and at least one pharmacodynamic model, an effect-site concentration for a drug to be administered.
2. A method according to clause 1, wherein the defining is performed for each drug to be administered.
3. A method according to clause 1 or 2, further comprising determining, based on the effect-site concentration of the drug and a pharmacokinetic model of the drug, a delivery rate for the drug.
4. A method according to any of clauses 1 to 3, wherein the determining is performed for each drug to be administered.
5. A method according to any of clauses 1 to 4, wherein the defining includes choosing, based on the at least one type of drug effect, at least one drug to be administered.
6. A method according to any of clauses 1 to 5, wherein the patient information indicates the weight and height of the patient.
7. A method according to any of clauses 1 to 6, wherein the patient information indicates the weight and height of the patient and the input information further includes drug information indicating at least one drug to be administered.
8. A method according to any of clauses 1 to 7, wherein the effect level information includes at least one effect level value within a predetermined effect scale.
9. A method according to any of clauses 1 to 8, wherein the at least one effect level value is one of a plurality of predetermined effect levels.
10. A method according to any of clauses 1 to 9, wherein the at least one pharmacodynamic model incorporates synergetic effects of at least two drugs.
11. A method according to any of clauses 1 to 10, further comprising administering at least one drug in response to the determining, wherein each of the at least one drug is administered at the delivery rate determined for that drug.
12. A method according to any of clauses 1 to 11, wherein the at least one type of drug effect belongs to a group including a hypnotic effect, an analgesic effect, and a neuromuscular blocking effect.
13. A method according to any of clauses 1 to 12, further comprising supplying the effect-site concentration as input information to a TCI pump.
14. A method according to any of clauses 1 to 13, further comprising providing a user interface module configured to display the predetermined effect scale and to enable a user to select the at least one effect level value from the predetermined effect scale.
15. A method according to any of clauses 1 to 14, wherein the user interface module is further configured to display at least one statistical reference curve indicating how the proportion of patients responding in a certain manner changes as the at least one effect level value changes.
16. An apparatus for controlling drug administration, the apparatus comprising
   - user interface means for receiving input information for drug administration, wherein the input information includes patient information and effect level information for at least one type of drug effect to be achieved in a patient;
   - computation means for defining, based on the input information and at least one pharmacodynamic model, an effect-site concentration for a drug to be administered.
17. An apparatus according to clause 16, wherein the computation means are configured to determine, based on the effect-site concentration of the drug and a pharmacokinetic model of the drug, a delivery rate for the drug.
18. An apparatus according to clause 16 or 17, wherein the computation means are configured to choose, based on the at least one type of drug effect, at least one drug to be administered.
19. An apparatus according to any of clauses 16 to 18 wherein the at least one pharmacodynamic model incorporates synergetic effects between at least two drugs.
20. An apparatus according to any of clauses 16 to 19, wherein the at least one type of drug effect belongs to a group including a hypnotic effect, an analgesic effect, and a neuromuscular blocking effect.
21. An apparatus according to any of clauses 16 to 20, wherein the computation means are further configured to supply the effect-site concentration as input information to a TCI pump.
22. An apparatus according to any of clauses 16 to 21, wherein the user interface means comprise a display unit configured to display a predetermined effect scale for the at least one type of drug effect, and wherein the user interface module is configured to enable a user of the apparatus to select at least one value from the predetermined effect scale.

## Claims

1. A method for controlling drug administration, the method comprising:
- receiving input information (401) for drug administration, wherein the input information includes patient information and effect level information for at least one type of drug effect to be achieved in a patient; and
- defining (403), based on the input information and at least one pharmacodynamic model, an effect-site concentration for a drug to be administered.

2. A method according to claim 1, wherein the defining (403) is performed for each drug to be administered.

3. A method according to any preceding claim, further comprising determining (404), based on the effect-site concentration of the drug and a pharmacokinetic model of the drug, a delivery rate for the drug.

4. A method according to any preceding claim, wherein the effect level information includes at least one effect level value within a predetermined effect scale.

5. A method according to claim 4, wherein the at least one effect level value is one of a plurality of predetermined effect levels.

6. A method according to any preceding claim, further comprising supplying the effect-site concentration as input information to a TCI pump (53a).

7. An apparatus (500) for controlling drug administration, the apparatus comprising
- user interface means (51,54) for receiving input information for drug administration, wherein the input information includes patient information and effect level information for at least one type of drug effect to be achieved in a patient;
- computation means (50) for defining, based on the input information and at least one pharmacodynamic model, an effect-site concentration for a drug to be administered.

8. An apparatus (500) according to claim 7, wherein the computation means (50) are configured to determine, based on the effect-site concentration of the drug and a pharmacokinetic model of the drug, a delivery rate for the drug.

9. An apparatus (500) according to claim 7 or 8, wherein the computation means (50) are further configured to supply the effect-site concentration as input information to a TCI pump (53a).

10. An apparatus (500) according to any of claims 7 to 10, wherein the user interface means (51,54) comprise a display unit (54) configured to display a predetermined effect scale for the at least one type of drug effect, and wherein the user interface module is configured to enable a user of the apparatus to select at least one value from the predetermined effect scale.
